(19) Europäisches Patentamt
European Patent Office
Office européen des brevets

(11) Publication number: 0 372 603 A2

(12) # EUROPEAN PATENT APPLICATION

(21) Application number: 89202886.1

(22) Date of filing: 14.11.89

(51) Int. Cl.5: A61K 7/22

(30) Priority: 15.11.88 US 271804

(43) Date of publication of application: 13.06.90 Bulletin 90/24

(84) Designated Contracting States: AT BE CH DE ES FR GB IT LI NL SE

(71) Applicant: Colgate-Palmolive Company
300 Park Avenue
New York, N.Y. 10022-7499(US)

(72) Inventor: Gaffar, Abdul
89 Carter Road
Princetown New Jersey(US)
Inventor: Collins, Elizabeth
45 Farm Road
St. James Head of the Harbor New York(US)

(74) Representative: Smulders, Theodorus A.H.J., Ir. et al
Vereenigde Octrooibureaux Nieuwe Parklaan 107
NL-2587 BP 's-Gravenhage(NL)

(54) **Antibacterial oral composition.**

(57) An antibacterial oral composition effective to promote oral hygiene containing an antibacterial antiplaque agent and an additive which substantially prevents staining of dental surfaces without substantially diminishing the antibacterial and antiplaque activity of the agent. Bis-biguanides such as chlorhexidine and alexidine, and quaternary ammonium salts, such as benzethonium chloride and cetyl pyridinium chloride are typical examples of antibacterial agents. The antistain additive is an aminoguanidine free base or water soluble salt thereof compatible with the bis-biguanide or quaternary ammonium salt.

EP 0 372 603 A2

# ANTIBACTERIAL ORAL COMPOSITION

This invention relates to an antibacterial oral composition which promotes oral hygiene.

Cationic nitrogen-containing antibacterial materials are well-known in the art. See, for instance the section on "Quaternary Ammonium and Related Compounds" in the article on "Antiseptics and Disinfectants" in Kirk-Othmer Encyclopedia of Chemical Technology, second edition (Vol. 2, p. 632-635), incorporated herein by reference. Cationic materials which possess antibacterial activity (i.e. are germicides) are used against bacteria and have been used in oral compositions to counter plaque formation caused by bacteria in the oral cavity.

Among the most common of these antibacterial antiplaque quaternary ammonium compounds is benzethonium chloride, also known as Hyamine 1622 or diisobutylphenoxy ethoxyethyl dimethyl benzyl ammonium chloride ("Hyamine" is a trademark). In an oral preparation this material is highly effective in promoting oral hygiene by reducing formation of dental plaque and calculus, which is generally accompanied by a reduction in caries formation and periodontal conditions. Other cationic antibacterial agents of this type are those mentioned, for instance, in U.S. Pat. Nos. 2,984,639; 3,325,402; 3,431,208; and 3,703,583; and British Pat. No. 1,319,396.

Other antibacterial antiplaque quaternary ammonium compounds include those in which one or two of the substituents on the quaternary nitrogen has a carbon chain length (typically alkyl group) of some 8 to 20, typically 10 to 18, carbon atoms while the remaining substituents have a lower number of carbon atoms (typically alkyl or benzyl group), such as 1 to 7 carbon atoms, typically methyl or ethyl groups. Dodecyl trimethyl ammonium bromide, dodecyl dimethyl (2-phenoxyethyl) ammonium bromide, benzyl dimethyl stearyl ammonium chloride, cetyl pyridinium chloride and quaternized 5-amino-1,3-bis (2-ethyl-hexyl)-5-methyl-hexa-hydropyrimidine are exemplary of other typical quaternary ammonium antibacterial agents.

Other types of cationic antibacterial agents which are desirably incorporated in oral compositions to promote oral hygiene by reducing plaque formation are the amidines such as the substituted bis-biguanides e.g. chlorhexidine and the corresponding compound, alexidine, having 2-ethylhexyl groups instead of chlorophenyl groups and other bi-biguanides such as those described in German patent application No. P 2,332,383 published Jan. 10, 1974, which sets forth the following formula:

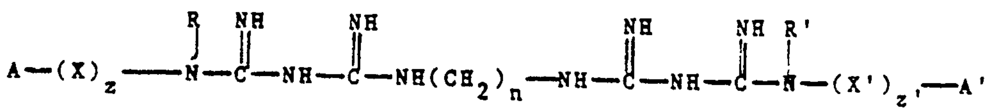

in which A and $A'$ signify, as the case may be either (1) a phenyl radical, which as substituent can contain up to 2 alkyl or alkoxy groups with 1 up to about 4 C-atoms, a nitro group or a halogen atom, (2) an alkyl group which contains 1 to about 12 C-atoms, or (3) alicyclic groups with 4 to about 12 C atoms, X and $X''$ as the case may represent an alkylene radical with 1-3 C atoms, z and $z'$ are as the case may be, either zero or 1, R and $R'$, as the case may be, may represent either hydrogen, an alkyl radical with 1 to about 12 C-atoms or an aralkyl radical with 7 to about 12 C-atoms, n is a whole number of 2 to inclusively 12 and the polymethylene chain ($CH_2$) can be interrupted by up to 5 ether, thioether, phenyl- or naphthyl groups; these are available as pharmaceutically suitable salts. Additional substituted bis-biguanidines are:

$N'$-(4-chlorobenzyl)-$N^5$-(2,4-dichlorobenzyl) biguanide;

p-chlorobenzyl biguanide,

4-chlorobenzhydryl guanylurea:

$N^3$-lauroxypropyl-$N^5$-p-chloro-benzyl biguanide;

5,6-dichloro-2-guanidobenzimidazole;

and

N-p-chlorophenyl-$N^5$-laurylbiguanide.

The long chain tertiary amines also prossess antibacterial and antiplaque activity. Such antibaterial agents include tertiary amines having one fatty alkyl group (typically 12 to 18 carbon atoms) and 2 poly-(oxyethylene) groups attached to the nitrogen (typically containing a total of from 2 to 50 ethenoxy groups per molecule) and salts thereof with acids and compounds of the structure:

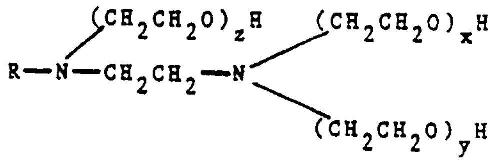

where R is a fatty alkyl group containing 12 to 18 carbon atoms and x, y and z total 3 or higher, as well as salts thereof. Another long chain tertiary amine is hexetidine. Generally, cationic agents are preferred for their antiplaque effectiveness.

The antibacterial antiplaque compound is preferably one which has an antibacterial activity such that its phenol co-efficient is well over 50, more preferably well above 100, such as above about 200 or more for S. aureus; for instance the phenol coefficient (A.O.A.C.) of benzethonium chloride is given by the manufacturer as 410, for S. aureus. The cationic antibacterial agent will generally be a monomeric (or possibly dimeric) material molecular weight well below 2,000, such as less than about 1,000. It is, however, within the broader scope of the invention to employ a polymeric cationic antibacterial agent. The cationic antibacterial is preferably supplied in the form of an orally acceptable salt thereof, such as the chloride, bromide, sulfate, alkyl sulfonate such as methyl sulfonate and ethyl sulfonate, phenylsulfonate, such as p-methylphenyl sulfonate, nitrate, acetate, gluconate, etc.

The nitrogen-containing cationic antibacterial agents and long chain tertiary amine antibacterial agents effectively promote oral hygiene, particularly by removing plaque. However, their use has been observed to lead to staining of dental surfaces or discoloration.

Human dental enamel contains a high proportion (about 95%) of hydroxyapatite (HAP) which includes $Ca^{+2}$ and $PO_4^{-3}$ ions. In the absence of dental plaque, additional $Ca^{+2}$ and $PO_4^{-3}$, particularly from saliva, can be deposited on the enamel and such deposits can include color bodies which could ultimately stain the tooth enamel as a calcified deposited thereon. It can be that as the cationic or long chain tertiary amine antibacterial agents remove plaque they also denature protein from saliva in the oral environment and the denatured protein can then act as a nucleating agent which is deposited on and stains or discolors tooth enamel.

Previously employed additives which reduced dental staining by cationic antibacterial antiplaque agents also generally reduced the activity of the antibacterial agents or its ability to act on dental plaque to measurable degrees. Further, Victamide (also known as Victamine C), an antinucleating agent, which is the condensation product of ammonia with phosphorus pentoxide actually increases staining even in the absence of a antibacterial antiplaque agent; and it and other known phosphorus containing agents such as disodiumethane-1-hydroxy-1,1-diphosphonic acid salt precipitate in the presence of antibacterial agent such as bis-biguanido compound, thereby reducing the antiplaque effectiveness of the antibacterial agent.

Other antinucleating agents have, however been successfully used to reduce staining in the presence of antibacterial antiplaque agent. Oral compositions containing these agents have been described in the patent literature, for example, in U.S. Patents 4,110,429; 4,118,472 to 4,118,476 inclusive 4,137,303 and 4,188,372 each to Gaffar et al (Colgate-Palmolive Company).

Nevertheless, the stain reduction ability of effective anti-nucleating agents is limited by the fact that while they retard growth of the color body or chromophore, they do not essentially prevent but rather or at least substantially delay its formation.

In the prior art particularly pertinent to development of the present invention efforts were made to reduce or prevent chromophore formation in the presence of an antibacterial antiplaque agent which tends to lead to staining or discoloration. Such efforts, entailing use of oxidizing or acylating agents are described in U.S. Patents 4,273,759 (peroxydiphosphate oxidizing agent); and U.S. Patent 4,080,441 (bis o-carboxyphenyl) ester of $C_{2-8}$ aliphatic dicarboxylic acid acylating agent).

The value of the aformentioned efforts, while important, have nevertheless been limited by chemical stability problems of the additives in formulated oral composition, including limited compatibility with many dental polishing agents when the oral composition is a dentifrice including such a component. Such problems could also be expected with reducing agents.

It is an advantage of this invention that an antibacterial oral composition is provided in which stain formation on dental surfaces is substantially prevented.

It is a further advantage of this invention that a stable antibacterial composition having little substantial tendency to stain surfaces, without merely delaying stain formation, is provided.

It is a further advantage of the invention that a cationic stain inhibitory additive is employed with an antibacterial antiplaque agent which may be cationic and which would otherwise tend to cause staining.

Other advantages will be apparent from consideration of the following disclosure.

In accordance with certain of its aspects, this invention relates to an oral composition comprising an orally acceptable vehicle, an effective amount of at least one cationic or long chain tertiary amine antibacterial antiplaque agent which tends to cause staining of dental surfaces and as an additive in amount which substantially prevents staining of dental surfaces, said additive being an aminoguanidine free base or water soluble salt thereof compatible with the antibacterial antiplaque agent.

Nitrogen-containing antibacterial agents which are cationic or long chain amine germicides which may be employed in the practice of this invention are described above. They are typically employed in amounts such that the oral product contains between about 0.001 and 15% by weight of the agent. Preferably for desired levels of antiplaque effect, the finished oral product contains about 0.01 to about 5%, and most preferably about 0.1 to 1.0% by weight of the antibacterial agent referring to its free base form, typically about 0.01 to 0.5% in a mouthrinse and about 0.5 to 1% in a dentifrice, topical solution or professionally administered prophylactic paste.

It is believed that the color chromophore may normally form on dental surfaces upon contact with the stain producing antibacterial anti-plaque agent by the following reaction (in the absence of stain preventing guanidine agent):

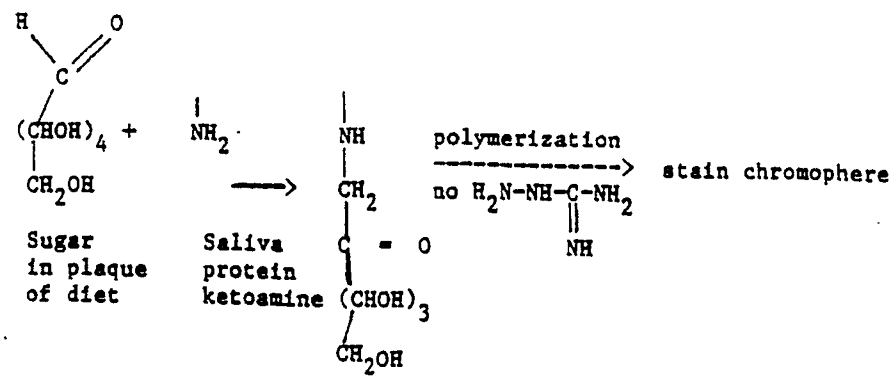


It is also believed that the formation of the color chromophore is substantially prevented by the presence of the stain preventing guanidine agent with the unreactive polymer formed by the above reactions thus:

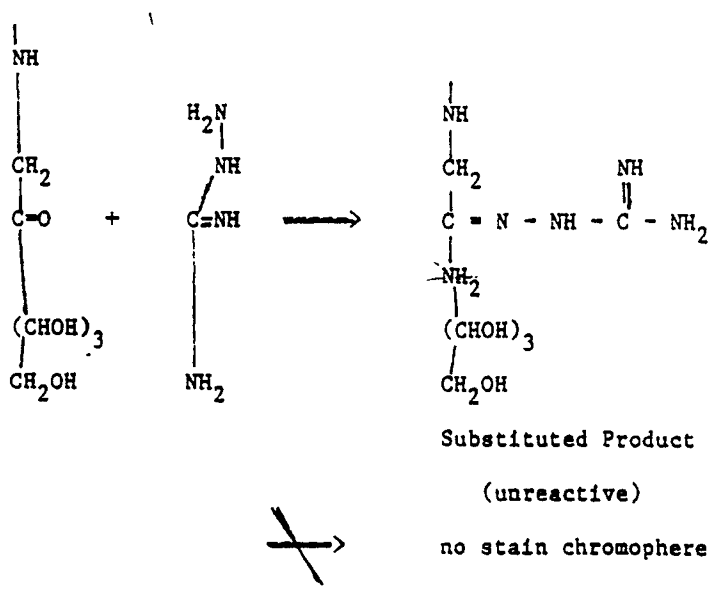


The following are examples of specific guanidine antistain agents which are compatible with the antibacterial antiplaque agent and which may be employed in the present invention:

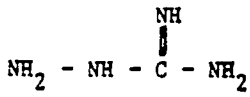
Hydrazine Carboximidamide (aminoguanidine)

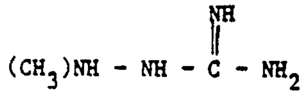
Methyl Hedrazine Carboximidamide

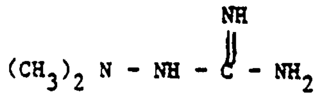
Dimethyl Hydrazine Carboximidamide

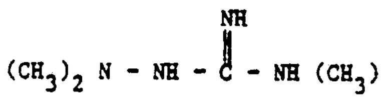
Dimethyl Hydrazine Carboximidmethylamide

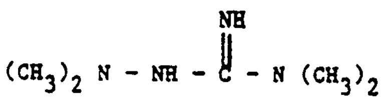
Dimethyl Hydrazine Carboximidmethylamide

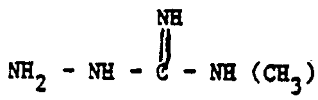
Hydrazine Carboximidmethylamide

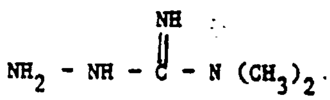
Hydrazine Carboximidmethylamide

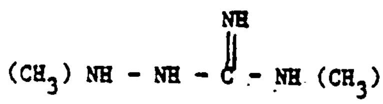
Methyl Hydrazine Carboximidmethylamide

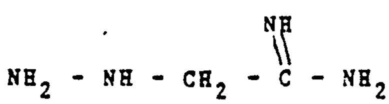
Hydrazine Acetylimidamide

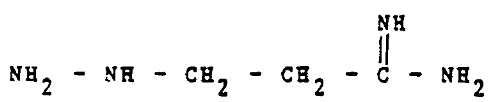
Hydrazine Propylimidamide

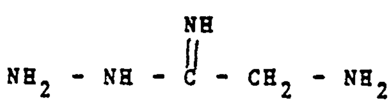
Hydrazine Carboximidmethyamine

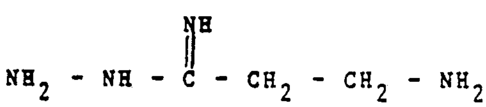
Hydrazine Carboimidethylamine

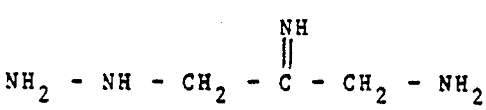
Hydrazineacetylimidmethylamide

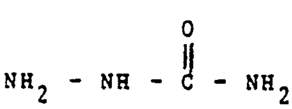
Hydrazine Carboxamide

In addition to the aminoguanidine free bases above, water soluble salts thereof, e.g. acetate, gluconate, lactate, propionate, formate, tartrate, etc. may be employed. The preferred compound is aminoguanidine, $H_2(N\text{-}NH\text{-}C\,(=NH)NH_2)$, free base or water soluble salt, particularly acetate and gluconate.

The concentration of the guanidine antistain additives in oral compositions can range widely, typically upward from about 0.01% by weight with no upper limit except as dictated by cost or possible

incompatibility with the vehicle. Generally, concentrations of about 0.01 to about 10% and preferably about 0.05 to 2% by weight are utilized. Oral compositions which in the ordinary course of usage could be ingested (e.g. liquids) preferably contain lower concentrations of these additives. Thus, a mouthwash in accordance with this invention preferably contains less than about 1% weight of the additive. Dentifrice compositions, topical solutons and prophylactic pastes, the latter to be administered professionally, preferably contain about 0.1 to 2% by weight of the additive. Most desirably the antistain additive is present in a molar ratio relative to the amount of antibacterial antiplaque agent (based on free base forms) of about 0.2:1 to about 6:1, preferably about 0.5:1 to about 4:1.

The stain which generally occurs on dental enamel is substantially prevented by reduced chromophore formation without essentially increasing over a prolonged period when the guanadine antistain additive and the antibacterial agent are employed. Moreover, oral preparations containing these materials are quite stable.

In certain highly preferred forms of the invention the oral composition may be substantially liquid in character, such as a mouthwash or rinse. In such a preparation the vehicle is typically a water-alcohol mixture. Generally, the ratio of water to alcohol is in the range of from about 1:1 to about 20:1 preferably from 3:1 to 20:1 and most preferably about 17:3, by weight. The total amount of water-alcohol mixture in this type of preparation is typically in the range of from about 70 to about 99.9% by weight of the preparation. The pH of such liquid and other preparations of the invention is generally in the range of from about 4.5 to about 9 and typically from about 5.5 to 8. The pH is preferably in the range of from about 6 to about 8.0. It is noteworthy that the compositions of the invention may be applied orally at a pH below 5 without substantially decalcifying dental enamel.

Such liquid oral preparations may also contain a surface active agent and/or a fluorine-providing compound.

In certain other desirable forms of this invention, the oral composition may be substantially solid or pasty in character, such as toothpowder, dental tablet, chewing gum lozenge, toothpaste or dental cream. The vehicle of such solid or pasty oral preparations contains polishing material. Examples of polishing materials are water-insoluble sodium metaphosphate, potassium metaphosphate, tricalcium phosphate, dihydrated calcium phosphate, anhydrous dicalcium phosphate, calcium pyrophosphate, magnesium orthophosphate, trimagnesium phosphate, calcium carbonate, alumina, hydrated alumina, aluminum silicate, zirconium silicates, silica, bentonite, and mixtures thereof. Preferred polishing materials include crystalline silica having particle sizes of up to 5 microns, a mean particle size of up to 1.1 microns, and a surface area of up to 50,000 $cm^2/gm$, silica gel, complex amorphorus alkali metal aluminosilicate and hydrated alumina.

Alumina, particularly the hydrated alumina sold by Alcoa as C333, which as an alumina content of 64.9% by weight, a silica content of 0.008%, a ferric oxide content of 0.003%, and a moisture content of 0.37%, at 110°C., and which has a specific gravity of 2.42 and a particle size such that 100% of the particles are less than 50 microns and 84% of the particles are less than 20 microns, is particularly desirable.

When visually clear gels are employed, a polishing agent of colloidal silica, such as those sold under the trademark SYLOID as Syloid 72 and Syloid 74 or under the trademark SANTOCEL as Santocel 100 and alkali metal aluminosilicate complexes are particularly useful, since they have refractive indices close to the refractive indices of gelling agent-liquid (including water and/or humectant) systems commonly used in dentifrices.

The polishing material is generally present in amounts ranging from about 20 to about 99% by weight of the oral preparation. Preferably, it is present in amounts ranging from about 20 to about 75% in toothpaste, and from about 70 to about 99% in toothpowder.

In the preparation of toothpowders, it is usually sufficient to admix mechanically, e.g., by milling, the various solid ingredients in appropriate quantities and particle sizes.

In pasty oral preparations the above-defined combination of the antibacterial antiplaque agent and antistain additive should be compatible with the other components of the preparation. Thus, in a toothpaste, the liquid vehicle may comprise water and humectant typically in an amount ranging from about 10 to about 90% by weight of the preparation. Glycerine, sorbitol, or polyethylene glycol may also be present as humectants comprise mixtures of water, glycerine and sorbitol.

In clear gels where the refactive index is an important consideration, about 3-30% by weight of water, 0 to about 80% by weight of glycerine, and about 20-80% by weight of sorbitol is preferably employed. A gelling agent, such as natural or synthetic gums or gum-like materials, typically Irish moss, sodium carboxymethylcellulose, methyl cellulose, or hydroxyethyl cellulose, may be employed. Other gelling agents which may be employed include gum tragacanth, polyvinlypyrrolidone and starch. They are usually present in toothpaste in an amount up to about 10% by weight, preferably in the range of from about 0.5 to about

5%. The preferred gelling agents are methyl cellulose and hydroxyethyl cellulose. In a toothpaste or gel, the liquids and solids are proportioned to form a creamy or gelled mass which is extrudable from a pressurized container or from a collapsible, e.g., aluminum or lead, tube.

The solid or pasty oral preparation which typically has a pH measured on a 20% slurry of about 4.5 to 9, generally about 5.5 to about 8 and preferably about 6 to about 8.0, may also contain a surface active agent and/or a fluorine-providing compound.

It will be understood that, as is conventional, the oral preparations, are to be sold or otherwise distributed in suitable labelled packages. Thus a jar of mouthrinse will have a label describing it, in substance, as a mouthrinse or mouthwash and having directions for its use; and a toothpaste will usually be in a collapsible tube, typically aluminum or lined lead, or other squeeze dispenser for metering out the contents, having a label describing it, in substance, as a mouthrinse or mouthwash and having directions for its use; and a toothpaste will usually be in a collapsible tube, typically aluminum or lined lead, or other squeeze dispenser for metering our the contents, having a label describing it, in substance, as a toothpaste or dental cream. Similar comments apply to topical solutions and prophylactic pastes administered professioanlly.

In oral compositions such as mouthrinses and toothpastes, a surfactant is often present, e.g. to promote foaming. It will be understood that it is preferable to employ nonionic or zwitter ionic surfactants rather than their anionic counterparts. Examples of water-soluble nonionic surfactants are condensation products of ethylene oxide with various reactive hydrogen-containing compounds reactive therwith having long hydrophobic chains e.g. aliphatic chains of about 12 to 20 carbon atims), which condensation products ("ethoxamers") contain hydrophilic polyoxyethylene moieties, such as condensation products of ethylene oxide with fatty acids, fatty alcohols, and fatty amides, Including alcohols such as sorbitan monostearate or polypropyleneoxide (that is Pluronic materials).

Betaine zwitterionic surfactants are also desirably used. Betaine surfactant has the general formula:

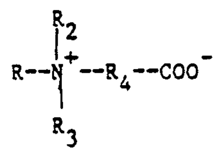

wherein $R_1$ is an alkyl group having 10 to about 20 carbon atoms, preferably 12 to 16 carbon atoms or the amido radical,

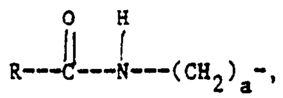

wherein R is an alkyl group having about 10 to 20 carbon atoms and a is the integer 1 to 3; $R_2$ and $R_3$ are each alkyl groups having 1 to 3 carbons and preferably 1 carbon; $R_4$ is an alkylene or hydroxyalkylene group having from 1 to 4 carbon atoms and, optionally, one hydroxyl group. Typical alkyldimethyl betaines include decyl betaine or 2-(N-decyl-N,N-dimethylammonio) acetate, coco betaine or 2-(N-coco-N,N-dimethylammonio)acetate, myristyl betaine, palmityl betaine, lauryl betaine, cetyl betaine, stearyl betaine, etc. The amidobetaines similarly include cocoamidoethyl betaine, cocoamidopropyl betaine, lauramidopropyl betaine and the like.

The betaines, which are zwitterionic materials, function as a foaming agent in the quaternary-containing dentifrice compositions. They act cationically over a wide pH range, but do not deactivate the quaternary antimicrobial activity.

In addition to the non-interference exhibited by the betaines with the quaternary activity, laboratory foam tests have shown that formulations containing both quaternary ammonium compound (quat) and the betaine, foam very well.

A fluorine-providing compound may also be present in the oral preparation. These compounds may be slightly soluble in water or may be fully water-soluble. They are characterized by their ability to release fluoride ions in water and by substantial freedom from reaction with other compounds of the oral preparation. Among these materials are inorgainc fluoride salts, such as soluble alkali metal, alkaline earth metal and heavy metal salts, for example, sodium fluoride, potassium fluoride, ammonium fluoride, lead fluoride, a copper fluoride such as cuprous fluoride, zinc fluoride, a tin fluoride such as stannic fluoride or

stannous chlorofluoride, barium fluoride, sodium fluorsilicate, ammonium fluorosilicate, sodium fluorozirconate, sodium monofluorophosphate, aluminum mono- and di-fluorophosphate, and fluorinated sodium calcium pyrophosphate. Alkali metal and tin fluorides, such as sodium and stannous fluorides, sodium monofluorophosphate and mixtures thereof, are preferred.

The amount of the fluorine-providing compound is dependent to some extent upon the type of compound, its solubility, and the type of oral preparation, but it must be a nontoxic amount. In a solid oral preparation, such as toothpaste or toothpowder, an amount of such compound which releases a maximum of about 1% by weight of the preparation is considered satisfactory. Any suitable minimum amount of such compound may be used, but it is preferable to employ sufficient compound to release about 0.005 to 1%. In the case of sodium monofluorophosphate, the compound may be present in an amount up to 7.6% by weight, more typically about 0.76%.

In a liquid oral preparation such as a mouthwash, the fluorine-providing compound is typically present in an amount sufficient to release up to about 0.13%, preferably about 0.0013 to 0.1% and most preferably about 0.0013 to 0.05%, by weight, of fluoride ion.

Various other materials may be incorporated in the oral preparations of this invention. Examples are whitening agents, preservatives, silicones, chlorophyll compounds, and ammoniated material such as urea, dimmonium phosphate, and mixtures thereof. These adjuvants, where present, are incorporated in the preparations in amounts which do not substantially adversely affect the properties and characteristics desired.

Any suitable flavoring or sweetening material may also be employed. Examples of suitable flavoring constituents are flavoring oils, e.g., oils of spearmint, peppermint, wintergreen, sassafras, clove, sage, euclayptus, marjoram, cinnamon, lemon, and orange, and methyl salicylate. Suitable sweetening agents include sucrose, lactose, maltose, sorbitol, sodium cyclamate, perillartine, and saccharine. Suitably, flavor and sweetening agents may together comprise from about 0.01 to 5% or more of the preparation.

In preparing the oral compositions of this invention comprising the above-defined combination of antibacterial agent and guanidine additive after the other ingredients (except perhaps some of the water) are mixed or contacted with each other to avoid a tendency for said agent to be precipitated.

For instance, a mouthrinse or mouthwash may be prepared by mixing ethanol and water with flavoring oil, nonionic surfactant, humectant, cationic antibacterial antiplaque agent, such as benzethonium chloride or chlorohexidine, sweetener, color and then the guanidine additive, followed by additional water as desired.

A toothpaste may be prepared by forming a gel with humectant, gum or thickener such as hydroxyethyl cellulose, sweetener and adding thereto polishing atent, flavor, antibacterial agent, such as benzethonium chloride or chlorhexidine, additional water, and then the guanidine additive. If sodium carboxymethyl cellulose is employed as the gelling agent the procedure of either U.S. Pat No. 3,842,168 or U. S. Pat No. 3,843,779, modified by the inclusion of the oligomer additive, is followed.

In the practice of this invention an oral composition according to this invention such as a mouthwash or toothpaste containing cationic or long chain amine antibacterial antiplaque agent in an amount effective to promote oral hygiene and the guanidine additive in an amount effective to reduce staining of dental surfaces otherwise resulting from the presence of the antibacterial antiplaque agent, is applied regularly to dental enamel, preferably from about 5 times per week to about 3 times daily.

The following specific examples are further illustrative of the nature of the present invention, but it is understood that the invention is not limited thereto. All amounts and proportions referred to herein and in the appended claims are by weight unless otherwise indicated.

## EXAMPLE 1

The following in vitro method correlates with and simulates the reaction whereby hydroxyapatite tooth mineral material is stained brown:

2.0 g hydroxyapatite (HAP), 10 ml of 25% Bovine Serum Albumin in 0.1M phosphate buffer (pH 7.0), 2.5 ml of 0.1M phosphate buffer containing mono-basic and di-basic sodium orthophosphate (pH 7), 25.0 ml of a test sample and 12.5 ml of 30% buffered acetaldehyde (pH 7) are mixed for 24 hrs. at 37°C. The HAP is filtered and dried at 37°C. The color retained on the HAP powder is qualified with a Gardner Color Difference Meter.

In the Tables below and in following Examples, "CHX" signifies chlorhexidine gluconate and "Ag" signifies aminoguanidine gluconate.

TABLE 1

| In Vitro Stain Retention | | | |
|---|---|---|---|
| Test Sample | Rd | Mean Rd | Rd* |
| A. Buffer Control | 57.50 56.04 | 56.77 | -- |
| B. Placebo (amino-quanidine gluconate) | 48.53 47.89 | 48.21 | 8.56 |
| C. 0.12% CHX Rinse | 39.40 40.27 | 39.83 | 16.94 |
| D. 0.12% CHX + 0.12% Ag Rinse | 47.42 49.96 | 48.69 | 8.08 |
| E. 0.12% CHX + 0.25% Ag Rinse | 50.01 49.77 | 49.89 | 6.88 |
| F. 0.12% CHX + 1.0% Ag Rinse | 48.26 47.89 | 48.07 | 8.7 |
| Rd = Reflectance Units | | | |

The results indicate that 0.12, 0.25 and 1.0% of aminoguanidine gluconate reduces the chlorhexidine induced stain at least to about the level of the placebo.

## EXAMPLE 2

The antibacterial effect of chlorhexidine in the presence of aminoguanidine is determined by exposing the following full strength rinses to Actinomyces viscosus cells for 60 seconds.

TABLE 2

| Sample | Average CFU x $10^6$ | Percent of Cells Killed |
|---|---|---|
| A. Water Control | 15.86 | -- |
| B. Placebo Rinse (Identify) | 24.83 | -- |
| C. CHX Rinse | 0 | 100% |
| D. CHX + 0.25% Ag Rinse | 0 | 100% |
| E. 0.25% Ag Rinse | 26.00 | -- |
| CFU means colony forming units | | |

The results indicate that 0.25% Aminoguanidine alone, (1) has no antibacterial effect and (2) it does not compromise antibacterial activity of chlorhexidine.

## EXAMPLE 3

The antiplaque effect of chlorhexidine in the presence of aminoguanidine is determined by rinsing in

vitro plaque which is formed by Actinomyces viscosus and strep mutans on extracted human teeth for 30 seconds once per day for three days with the full strength rinses below. The plaque growth is induced on teeth by daily transferring the teeth to an inoculated fresh medium (tryptocase soy broth). The amount of plaque is scored visually as well as after disclosing with erythrosine dye. The dye is then extracted with ethanol. The amount of the dye adsorbed is directly proportional to the amount of the plaque on teeth. The dye concentration is measured spectrophotometrically at wavelength 533nm.

TABLE 3

| Sample | Visual Scores of Plaque (3 days) (Mean of 5 Tests) | Disclosed Plaque Extracts (533nm) | Percent Decrease in Plaque |
|---|---|---|---|
| A. Water Control | 3.125 | 0.176 | -- |
| B. Placebo + 0.25% Ag | 3.125 | 0.179 | -- |
| C. CHX Rinse | 1.0 | 0.023 | 87.15% |
| D. CHX + 0.25% Ag | 1.125 | 0.027 | 84.9% |
| E. CHX (0.12%) in $H_2O$ | 1.375 | 0.041 | 76.9% |

The results indicate that 0.25% Aminoguanidine alone (1) has no antiplaque activity and (2) does not compromise the antiplaque activity of chlorhexidine.

The observations set forth in Examples 1-3 lead to the conclusion that under the conditions set forth, aminoguanidine substantially reduces stain formation caused by chlorhexidine without diminishing the antibacterial and antiplaque effects of chlorhexidine, even though aminoguanidine is, itself, not an antibacterial or antiplaque material.

## EXAMPLE 4

1.0% of Aminoguanidine bicarbonate is dissolved in 100cc of distilled water. The pH is adjusted from 9 to pH 4 with gluconic acid. This solution is then mixed with 110-115 g of Anion Exchange Resin (AGI-$X_2$) (Acetate) for 30 min. at room temperature. The converted Aminoguanidine acetate is then collected via filtration (Whatman #1 filter). This filtrate is then used as solution "C" below.

| | Rinse Composition | Parts |
|---|---|---|
| A. | 95% Ethanol | 9.48 |
| | Glycerine, 99.3% Pure | 8.0 |
| | Flavor | 0.08 |
| B. | Deionized water | 40.0 |
| | Polyethylene Glycol-40 sorbitan (PEG 40) diisostearate | 0.2 |
| | Chlorhexidine digluconate | 0.12 |
| | Sodium Saccharin | 0.01 |
| C. | Aminoguanidine gluconate | 1.0 |
| | Deionized water | 40.6 |

Solutions A, B and C are prepared separately (pH 6.5-7). Solution C is added to Solution B and the resulting solution is added to Solution A. The final pH is 5.7 - 6.0. The rinse is stored in brown bottles.

| EXAMPLE 5 | | |
|---|---|---|
| Dentifrice Composition | | |
| | Parts | |
| | A | B |
| Hydroxyethylcellulose (Natrosol 250 MR) | 1.0 | 1.0 |
| Glycerine | 20.0 | 20.0 |
| Hydrated Alumina | 52.0 | 52.0 |
| Sodium saccharin | 0.2 | 0.2 |
| Chlorhexidine digluconate | 1.0 | 1.0 |
| Aminoguanidine gluconate | 2.0 | 5.0 |
| Betaine | 0.75 | 4.0 |
| Flavor | 1.0 | 1.0 |
| Sodium Benzoate | 0.5 | 0.5 |
| Water | Q.S. to 100 | Q.S. to 100 |

## EXAMPLE 6

## LOZENGE

75-80% Sugar
1-20% Corn Syrup
0.1-1.0 Flavor
1% Chlorhexidine Digluconate
2% Aminoguanidine Gluconate
1 to 5% Magnesium Stearate Lubricant
0.01 to 0.2% Water

| EXAMPLE 7 | |
|---|---|
| CHEWING GUM | PARTS |
| Gum base | 25.00 |
| Sorbitol (70%) | 17.00 |
| Chlorhexidine Digluconate | 1.80 |
| Aminogluamine Gluconate | 2.00 |
| NaF | 0.05 |
| Glycerine | 0.50 |
| Cryatalline Sorbitol | 53.00 |
| Flavor and Water | Q.S. to 100.00 |

## EXAMPLE 8

Adult squirrel monkeys are prepared for a gingivitis/plaque/stain evaluation including thorough prophylaxis. 36 surfaces (maxillary and mandibular; mesial, buccal and distal) are initially evaluated for plaque by a modified Quigley-Hein index and gingival inflation by the following scale:
0 - clinically normal; pink, form, knife-like gingival margin, triangular interdental papilla;

1 - change in the form of gingiva; gingival margin may be slightlyenlarged, no longer knife-like and firm, no color change;

2 - slight color change of marginal gingiva of interdental papilla, discrete areas of redness at most cornal surfaces of the free gingiva;

3- obvious color change in marginal gingiva or interdental papilla, no bleeding on probing;

4 - obvious color change; redness obvious; in addition, bleeding on probing or spontaneous bleeding.

The squirrel monkeys are then divided into 3 groups of four and each is treated in a double blind manner once daily for 6 weeks. All dentitions are sprayed for 30 seconds from polyethylene bottles containing rinse solution.

Initial and 6 week evaluations for three studies for plaque formation and gingival inflamation and 6 week evaluations for stain (indicate general protocol) follow, with stain

TABLE 4

| CHLORHEXIDINE/STAIN QUENCHER TECHNOLOGY-AMINOGUANIDINE | | | | | | | |
|---|---|---|---|---|---|---|---|
| OBJECTIVE: Prevent/Reduce Chlorhexidine Induced Staining | | | | | | | |
| In Vivo Efficacy of CHX/AG in NonHuman Primates | | | | | | | |
| Study# | Treat. | Initial Gingival Index | Final Gingival Index | Initial Plaque Index | Final Plaque Index | Initial Stain Index | Final Stain Index |
| A. | Control | 0.44 | 0.65 | 0.63 | 0.61 | --- | --- |
| | CHX | 0.43 | 0.49 | 0.55 | 1.24 | --- | --- |
| | CHX/0.25%AG | 0.55 | 0.41 | 0.41 | 0.95 | --- | --- |
| B. | CHX | 0.54 | 0.47 | 0.15 | 0.47 | 0.01 | 0.62 |
| | CHX/0.50%AG | 0.51 | 0.40 | 0.19 | 0.53 | 0.04 | 0.35 (41.5% reduction) |
| C. | Control | 0.55 | 0.51 | 0.25 | 0.51 | | |
| | CHX | 0.56 | 0.63 | 0.53 | 0.83 | | |
| | CHX/1.1%AG | 0.60 | 0.59 | 0.50 | 0.81 | | |
| CONCLUSIONS: | | | | | | | |
| 1. Aminoguanidine does not appear to compromise the efficacy of chlorhexidine<br>2. Aminoguanidine reduces staining by approximately 60% | | | | | | | |

This invention has been disclosed with respect to preferred embodiments and it will be understood that modifications and variations thereof obvious to those skilled in the art are to be included within the spirit and purview of this application and the scope of the appended claims.

## Claims

1. An oral composition comprising an orally acceptable vehicle, an effective amount of at least one cationic or long chain tertiary amine antibacterial antiplaque agent which tends to cause staining of dental surfaces and an additive in amount which substantially prevents staining of dental surfaces, said additive being an aminoguanidine free base or water soluble salt thereof compatible with said antibacterial antiplaque agent.

2. The oral composition claimed in Claim 1 wherein said antibacterial antiplaque agent is present in amount of about 0.001-15% by weight and said additive is present in amount of about 0.01-10% by weight and the molar ratio of the antistain additive to the antibacterial antiplaque additive is from about 0.2:1 to about 6:1.

3. The oral composition claimed in Claim 2 wherein said antibacterial antiplaque agent is a cationic nitrogen-containing antibacterial agent.

4. The oral composition claimed in Claim 3 wherein said cationic nitrogen-containing antibacterial agent is chlorhexidine free base or salt thereof.

5. The oral composition claimed in Claim 2 wherein said additive is selected from the group consisting of:

Hydrazine Carboximidamide (aminoguanidine)
Methyl Hydrazine Carboximidamide
Dimethyl Hydrazine Carboximidamide
Dimethyl Hydrazine Carboximidimethylamide
Dimethyl Hydrazine Carboximidimethylamide
Hydrazine Carboximidmethylamide
Hydrazine Carboximidmethylamide
Methyl Hydrazine Carboximidimethylamide
Hydrazine Acetylimidamide
Hydrazine Propylimidamide
Hydrazine Carboximidmethylamine
Hydrazine Carboimidethylamine
Hydrazineacetylimidmethylamide
Hydrazine Carboxamide

6. The oral compostion claimed in Claim 5 wherein said additive is aminoguanidine or water soluble salt thereof.

7. The oral composition claimed in Claim 6 wherein said additive is aminoguanidine gluconate.

8. The oral composition of Claim 1 wherein said vehicle is an aqueous-alcohol and said composition is a mouthwash.

9. The oral composition of Claim 1 wherein said vehicle comprises a liquid vehicle and a gelling agent and a dentally acceptable polishing material is present and said composition is a toothpaste.